# EUROPÄISCHE PATENTSCHRIFT

(11) **EP 3 712 230 B1**
(45) Veröffentlichungstag und Bekanntmachung des Hinweises auf die Patenterteilung: **17.05.2023**
(21) Anmeldenummer: 20163215.5
(22) Anmeldetag: 16.03.2020
(51) Int. Cl.: C09K 19/30, C09K 19/34

(54) **FLÜSSIGKRISTALLINE VERBINDUNGEN**
LIQUID CRYSTAL COMPOUNDS
COMPOSÉS À CRISTAUX LIQUIDES

(30) Priorität: 18.03.2019 DE 102019001887
(43) Veröffentlichungstag der Anmeldung: 23.09.2020
(73) Patentinhaber: Merck Patent GmbH, 64293 Darmstadt (DE)
(72) Erfinder: Brocke, Constanze, 64521 GROSS-GERAU (DE); Linke, Katharina, 63110 RODGAU (DE); Laut, Sven, 64331 WEITERSTADT (DE)

(56) Entgegenhaltungen:
- EP-A1- 3 260 450
- WO-A1-2004/106460
- WO-A1-2018/141759

## Beschreibung

Die Erfindung betrifft Verbindungen der Formel I wie unten definiert, die als Strukturelement eine Kombination aus einem 1,4-substituierten Cyclohexenring neben einem Dioxan- oder Tetrahydropyranring und einer substituierten Biphenylgruppe aufweisen. Die Erfindung umfasst daneben ein Verfahren zur Herstellung, flüssigkristalline Medien enthaltend mindestens eine Verbindung der Formel I sowie ihre Verwendung als Komponente(n) in flüssigkristallinen Medien. Darüber hinaus betrifft die vorliegende Erfindung Flüssigkristall- und elektrooptische Anzeigeelemente, welche die erfindungsgemäßen, flüssigkristallinen Medien enthalten.

In den vergangenen Jahren wurden die Anwendungsgebiete für flüssigkristalline Verbindungen auf verschiedene Arten von Anzeigevorrichtungen, elektrooptische Geräte, elektronische Komponenten, Sensoren, etc. erheblich ausgeweitet. Aus diesem Grund wurden eine Reihe verschiedener Strukturen vorgeschlagen, insbesondere auf dem Gebiet der nematischen Flüssigkristalle. Die nematischen Flüssigkristallmischungen haben bisher die breiteste Anwendung in flachen Anzeigevorrichtungen gefunden. Sie wurden besonders in passiven TN- oder STN-Matrixanzeigen oder Systemen mit einer TFT-Aktivmatrix eingesetzt.

Die erfindungsgemäßen, flüssigkristallinen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (electrically controlled birefringence), dem IPS-Effekt (in-plane switching) oder dem Effekt der dynamischen Streuung beruhen.

Die Verwendung von polaren Dioxan- und Tetrahydropyranverbindungen mit vier Ringen als flüssigkristalline Substanzen ist dem Fachmann nicht unbekannt. Es wurden bereits verschiedene Verbindungen mit einem O-heterocyclischen Ring als flüssigkristallines oder mesogenes Material und dessen Herstellung beschrieben, wie z. B. in den Druckschriften WO 2004/106460 A1 und EP 3260450 A1. Die darin vorgeschlagenen Verbindungen besitzen keinen Cyclohexenring. Als polare Endgruppe enthalten die Verbindungen z. B. -OCF₃ oder Fluor.

Cyclohexenverbindungen mit einem Dioxanring sind bekannt aus WO 2018/141759 A1, besitzen aber insgesamt nur drei Ringgruppen.

Der vorliegenden Erfindung lag die Aufgabe zugrunde, neue stabile Verbindungen aufzufinden, die als Komponente(n) flüssigkristalliner Medien geeignet sind. Insbesondere sollen die Verbindungen gleichzeitig eine vergleichsweise geringe Viskosität, sowie eine hohe dielektrische Anisotropie besitzen. Für viele aktuelle Mischungskonzepte im Bereich der Flüssigkristalle ist es vorteilhaft, Verbindungen mit einer positiven dielektrischen Anisotropie Δε in Verbindung mit einer mittleren bis hohen optischen Anisotropie zu verwenden.

Im Hinblick auf die verschiedensten Einsatzbereiche derartiger Verbindungen mit hohem Δε war es wünschenswert, weitere Verbindungen, vorzugsweise mit hohem Klärpunkt und geringer Viskosität zur Verfügung zu haben, die auf die jeweiligen Anwendungen genau maßgeschneiderte Eigenschaften aufweisen.

Der Erfindung lag somit als eine Aufgabe zugrunde, neue stabile Verbindungen aufzufinden, die als Komponente(n) flüssigkristalliner Medien, insbesondere für z. B. TN-, STN-, IPS-, FFS- und TN-TFT-Displays, geeignet sind.

Darüber hinaus sollten die erfindungsgemäßen Verbindungen unter den in den Anwendungsgebieten vorherrschenden Bedingungen thermisch und photochemisch stabil sein. Als Mesogene sollten sie eine breite nematische Phase in Mischungen mit flüssigkristallinen Cokomponenten ermöglichen sowie hervorragend mit nematischen Basismischungen, insbesondere bei tiefen Temperaturen, mischbar sein. Ebenso bevorzugt sind Substanzen mit einem niedrigen Schmelzpunkt und einer geringen Schmelzenthalpie, da diese Größen wiederum Anzeichen für die oben genannten wünschenswerten Eigenschaften sind, wie z. B. eine hohe Löslichkeit, eine breite flüssigkristalline Phase und eine geringe Neigung zur spontanen Kristallisation in Mischungen bei tiefen Temperaturen. Gerade die Löslichkeit bei tiefer Temperatur unter Vermeidung von jeglicher Kristallisation ist wichtig für den sicheren Betrieb und Transport von Anzeigen in Fahr- und Flugzeugen und im Freien.

Überraschenderweise wurde gefunden, dass die erfindungsgemäßen Verbindungen vorzüglich als Komponenten flüssigkristalliner Medien geeignet sind. Mit ihrer Hilfe lassen sich flüssigkristalline Medien für Displays erhalten, die besonders hohe dielektrische Anisotropien benötigen, insbesondere für IPS- oder FFS-Anzeigen, aber auch für TN- oder STN-Anzeigen. Die erfindungsgemäßen Verbindungen sind hinreichend stabil und farblos. Besonders zeichnen sie sich durch hohe dielektrische Anisotropien (Δε) aus, aufgrund derer in der Anwendung in optischen Schaltelementen geringere Schichtdicken und damit niedrigere Schwellenspannungen erforderlich sind. Sie sind für Verbindungen mit vergleichbaren Eigenschaften gut löslich. Darüber hinaus weisen die erfindungsgemäßen Verbindungen einen vergleichsweise sehr hohen Klärpunkt sowie gleichzeitig niedere Werte für die Rotationsviskosität auf. Die Verbindungen besitzen relativ niedrige Schmelzpunkte. Mit den erfindungsgemäßen Verbindungen ist es überaschenderweise möglich, flüssigkristalline Mischungen mit hohen Werten für die elastischen Konstanten (K¹¹/K²²/K³³) herzustellen, ohne die übrigen Anwendungsparameter nachteilig zu beeinflussen. Daraus resultieren Medien mit geringer Schaltzeit und hohem Kontrast.

Mit der Bereitstellung der erfindungsgemäßen Verbindungen wird ganz allgemein die Palette der flüssigkristallinen Substanzen, die sich unter verschiedenen anwendungstechnischen Gesichtspunkten zur Herstellung flüssigkristalliner Mischungen eignen, erheblich verbreitert.

Die erfindungsgemäßen Verbindungen besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind. Es können aber auch den erfindungsgemäßen Verbindungen der Formel I flüssigkristalline Basismaterialien aus anderen Verbindungsklassen zugesetzt werden, um beispielsweise die dielektrische und/oder die optische Anisotropie eines solchen Dielektrikums zu beeinflussen und/oder um dessen Schwellenspannung und/oder dessen Viskosität zu optimieren.

Gegenstand der Erfindung sind somit Verbindungen der Formel I, 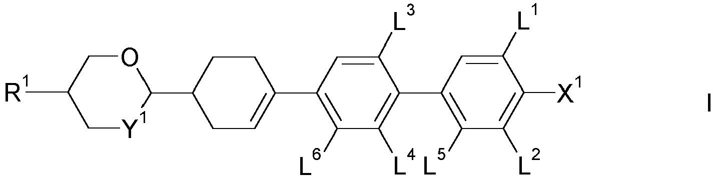 worin
- X¹: F, CF₃, OCF₃, OCHF₂, CHF₂, SCN oder CN,
- Y¹: O oder CH₂,
- R¹: Alkyl oder Alkenyl mit bis zu 8 Kohlenstoffatomen,
- L¹ und L²: unabhängig voneinander H oder F, bevorzugt F,
- L³: H oder F, bevorzugt F,
- L⁴: H oder F, bevorzugt H, und
- L⁵ und L⁶: unabhängig voneinander H oder CH₃, bevorzugt H, bedeuten.

Gegenstand der Erfindung ist weiterhin die Verwendung der Verbindungen der Formel I in flüssigkristallinen Medien.

Ebenfalls Gegenstand der vorliegenden Erfindung sind flüssigkristalline Medien mit mindestens zwei flüssiakristallinen Komponenten, welche mindestens eine Verbindung der Formel I enthalten.

Die Verbindungen der Formel I sind in reinem Zustand farblos und bilden für sich oder in Mischungen flüssigkristalline Mesophasen in einem für die elektrooptische Verwendung günstig qeleqenen Temperaturbereich. Mit den erfindungsgemäßen Verbindungen lassen sich breite nematische Phasenbereiche erzielen. In flüssigkristallinen Mischungen erhöhen die erfindungsgemäßen Substanzen die optische Anisotropie deutlich und/oder führen zu einer Verbesserung der Tieftemperatur-Lagerstabilität gegenüber veraleichbaren Mischunaen mit hoher dielektrischer Anisotropie. Gleichzeitig zeichnen sich die Verbindungen durch gute UV-stabilität aus.

Der Rest R¹ der Formel I und ihrer Unterformeln bedeutet bevorzugt einen geradkettigen Alkylrest mit 1 bis 7 C-Atomen oder einen unverzweigten Alkenylrest mit 2 bis 8 C-Atomen. insbesondere unverzweiates Alkyl mit 2 bis 7 C-Atomen.

Der Rest X¹ der Formel I bedeutet bevorzugt F, CF₃, OCF₃ oder -SCN, besonders bevorzugt F, CF₃ oder OCF₃, und ganz besonders bevorzugt F. Verbindungen der Formel I mit verzweigten oder substituierten Flügelgruppen R¹ können gelegentlich wegen einer besseren Löslichkeit in den üblichen flüssigkristallinen Basismaterialien von Bedeutung sein. Bevorzugt ist die Gruppe R¹ geradkettig.

Bevorzugt sind Verbindungen der Formeln I, worin L¹ F bedeutet und L² H oder F, insbesondere worin L¹ und L² F bedeuten. Weiterhin sind Verbindungen bevorzugt worin L³ H oder F bedeutet und L⁴ H, insbesondere L³ = H und L⁴ = F. Weiterhin bevorzugt sind Verbindungen worin L⁶ H und L⁵ H oder Methyl bedeutet, insbesondere L⁵ und L⁶ = H.

Der Rest R¹ ist besonders bevorzugt ausgewählt aus den Teilstrukturen:
- CH₃
- C₂H₅
- C₃H₇
- C₄H₉
- C₅H₁₁
- C₆H₁₃
- CH=CH₂
- CH=CH₂-CH₃
- CH₂-CH₂-CH=CH₂
- CH₂-CH₂-CH=CH-CH₃
worin die Alkylketten vorzugsweise unverzweigt sind (n-Alkyl).

Besonders bevorzugte Verbindungen der Formel I sind die Verbindungen der Formeln I-1 bis I-6: 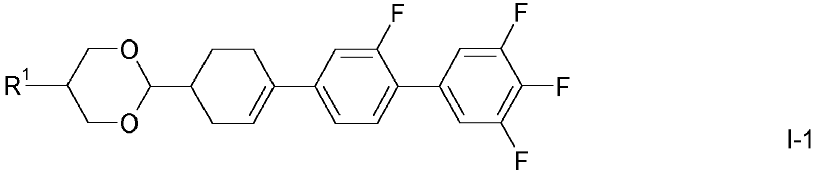 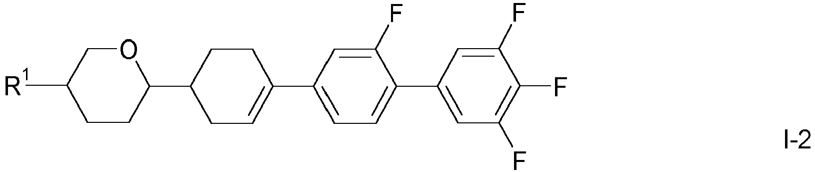 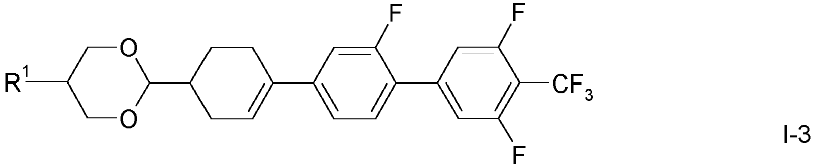 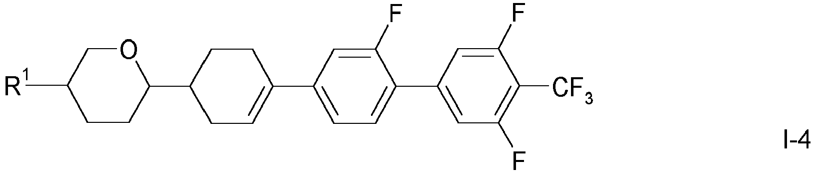 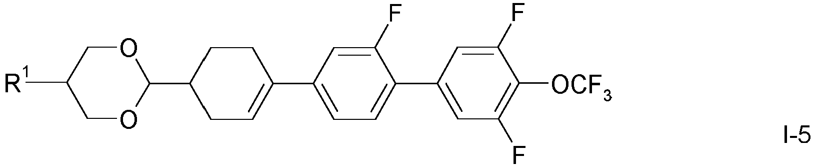 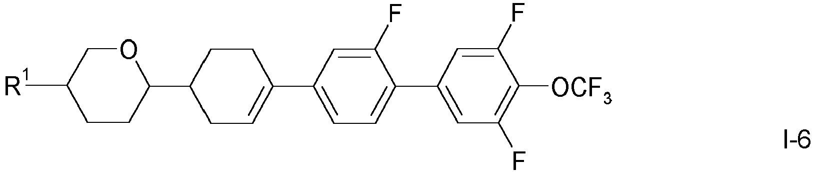 worin R¹ unabhängig die oben angegebenen Bedeutungen hat. Unter den Verbindungen der Formeln I-1 bis I-6 sind die Verbindungen der Formeln I-1 und I-2 bevorzugt. R¹ bedeutet besonders bevorzugt eine n-Alkylgruppe mit 2, 3, 4, 5, 6 oder 7 C-Atomen.

Beispielhafte Verbindungen sind die Folgenden:

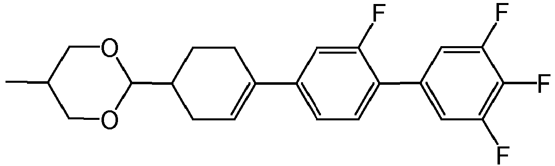

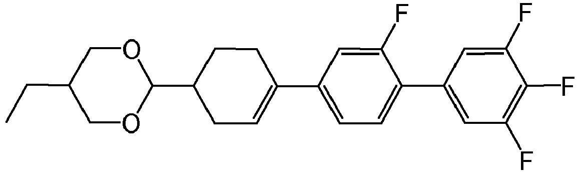

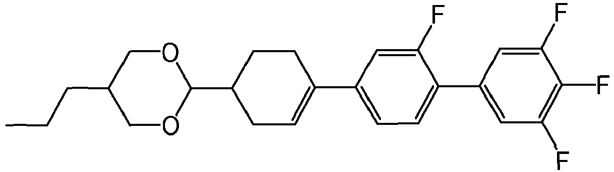

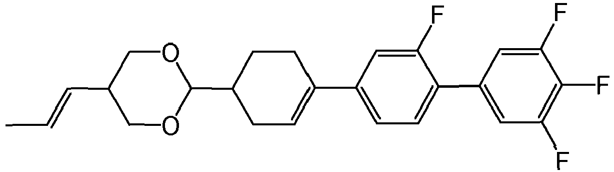

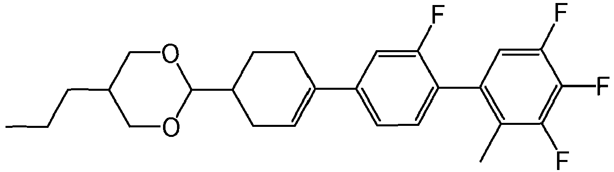

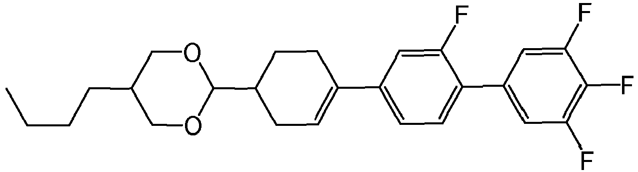

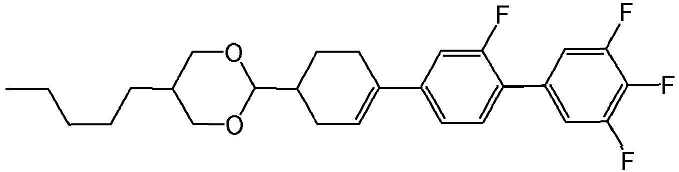

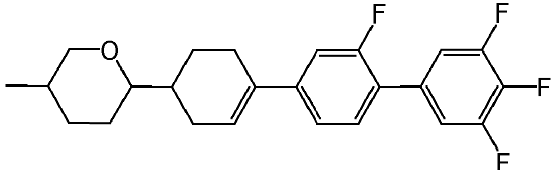

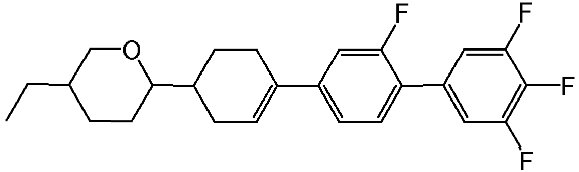

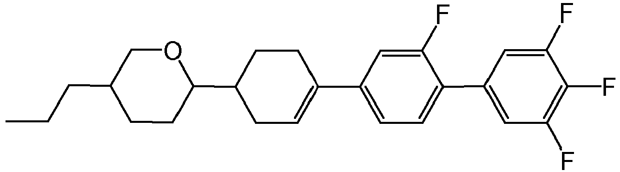

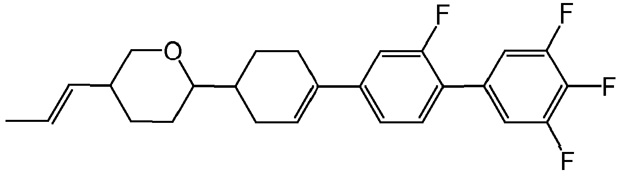

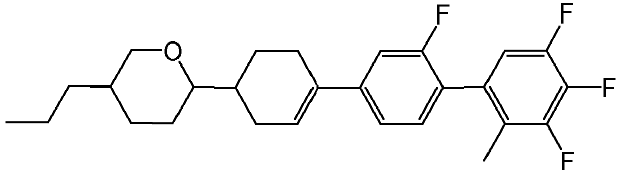

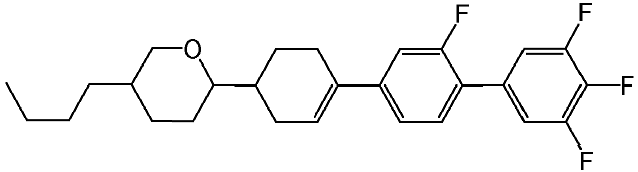

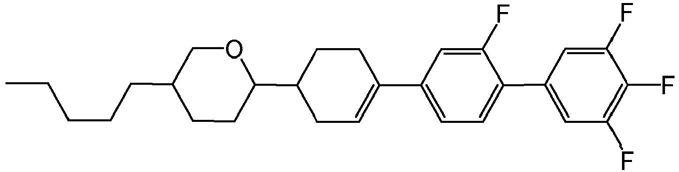

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorteilhafterweise wie an der folgenden beispielhaften Synthese und den Beispielen ersichtlich hergestellt werden (Schema 1 bis 3):

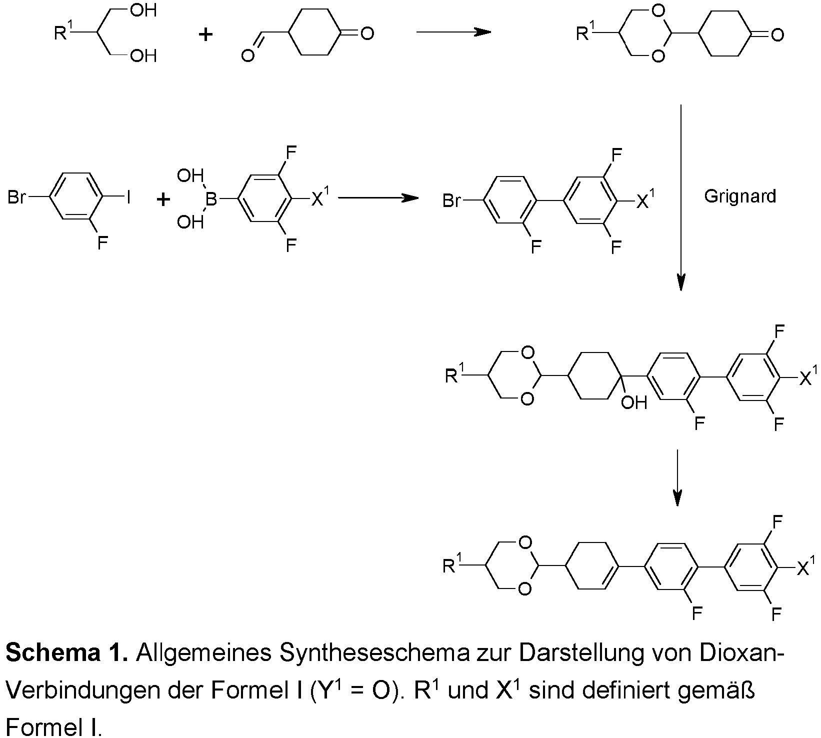

Durch Abwandlung der Reaktionsabfolge aus Schema 1 ergibt sich alternativ das Syntheseschema 2 zur Herstellung von entsprechenden Tetrahydropyranverbindungen.

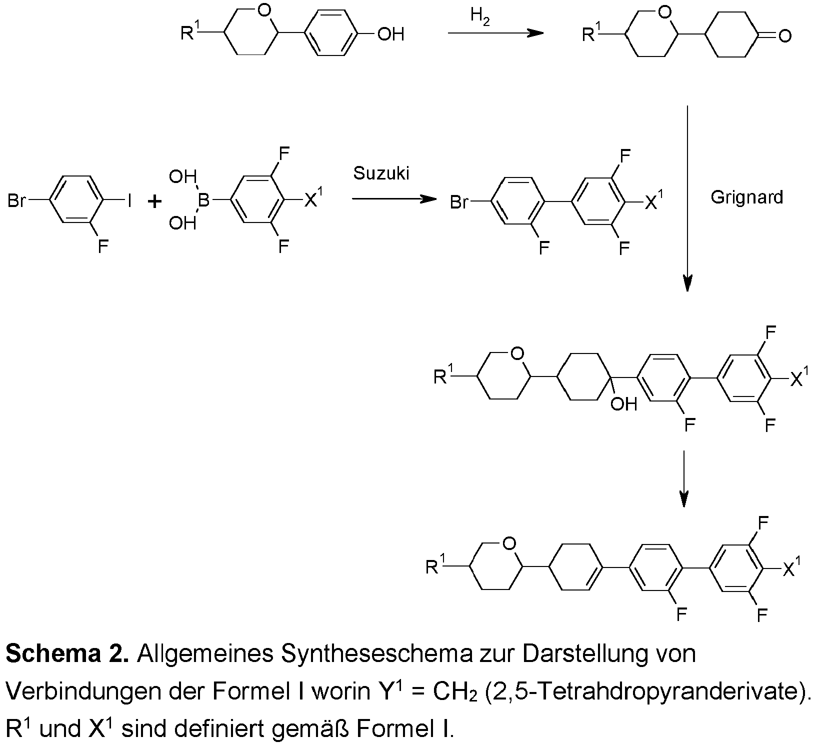

Entsprechende Ausgangsprodukte lassen sich in der Regel vom Fachmann ohne Weiteres über literaturbekannte Synthesemethoden herstellen oder sind kommerziell erhältlich.

Anstelle der skizzierten Grignard-Verbindungen nach Schema 1 und 3 können auch Aryllithiumverbindungen verwendet werden, die analog durch Halogen-Metall-Austausch mit Alkyllithium-Verbindungen bei tiefen Temperaturen zugänglich sind (vgl. z.B. US 4,940,822).

Die Erfindung hat daher auch ein Verfahren zur Herstellung von Verbindungen der Formel I zum Gegenstand das einen Verfahrensschritt umfasst, in dem eine Arylhalogenverbindung der Formel II 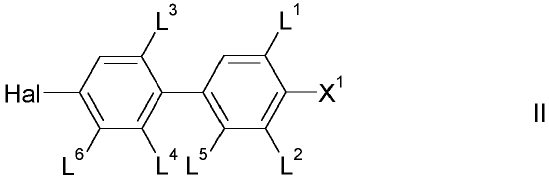
worin X¹, L¹, L², L³ und L⁴, wie für Formel I definiert sind, und
Hal Br, I oder C!, bevorzugt Br, bedeutet, mit einer Verbindung der Formel III 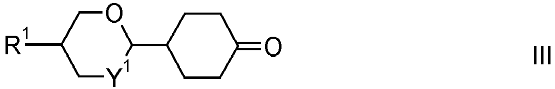 worin
R¹ und Y¹ wie für Formel I definiert sind,
zu einer Verbindung der Formel IV umgesetzt wird, 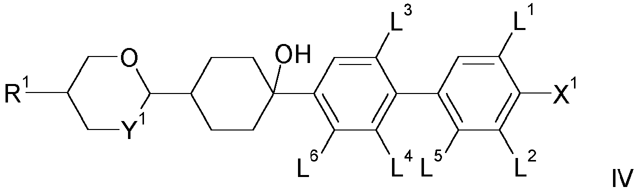
und weiter zu einer Verbindung der Formel I 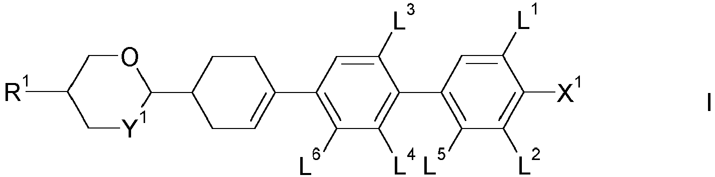
worin die Gruppen wie vorangehend definiert sind,
umgesetzt wird.

Zur Reaktion von Verbindung II mit III wird die Verbindung II in der Regel zunächst metalliert, beispielsweise durch Umsetzung mit Alkylmagnesiumhalogeniden, Magnesium oder Lithiumalkylverbindungen. Anschließend wird die Alkylmetallverbindung mit dem Cyclohexylketon III umgesetzt. Das Cyclohexanol-Produkt der Formel IV wird unter Säurekatalyse durch Wasserabscheidung zum Cyclohexen der Formel I umgesetzt.

Die verwendeten Reaktionsmethoden und Reagenzien sind prinzipiell literaturbekannt. Weitere Reaktionsbedingungen können den Ausführungsbeispielen entnommen werden.

Weitere, vorangehend nicht genannte, bevorzugte Verfahrensvarianten lassen sich den Beispielen oder den Ansprüchen entnehmen.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

In der vorliegenden Offenbarung bedeutet der 2,5-disubstituierte Dioxanring der Formel 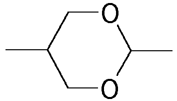 bevorzugt einen 2,5-trans-konfigurierten Dioxanring, d.h. die Substituenten stehen in der bevorzugten Sesselkonformation beide bevorzugt in der äquatorialen Position. Ebenso bedeutet der 2,5-disubstituierte Tetrahydropyranring der Formel 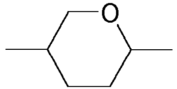 bevorzugt einen 2,5-trans-konfiguriertenTetrahydropyranring, d.h. die Substituenten stehen in der bevorzugten Sesselkonformation beide bevorzugt in der äquatorialen Position.

Gegenstand der Erfindung sind auch flüssigkristalline Medien enthaltend eine oder mehrere der erfindungsgemäßen Verbindungen der Formel I. Die flüssigkristallinen Medien enthalten wenigstens zwei Komponenten. Man erhält sie vorzugsweise indem man die Komponenten miteinander vermischt. Ein erfindungsgemäßes Verfahren zur Herstellung eines flüssigkristallinen Mediums ist daher dadurch gekennzeichnet, dass man mindestens eine Verbindung der Formel I mit mindestens einer weiteren mesogenen Verbindung vermischt und gegebenenfalls Additive zugibt.

Die erzielbaren Kombinationen aus Klärpunkt, Viskosität bei tiefer Temperatur, thermischer/UV-Stabilität, dielektrischer Anisotropie, Schaltzeit und Kontrast übertreffen bei weitem bisherige Materialien aus dem Stand der Technik.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexane, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-COO-E-R" 2

R'-L-CF₂O-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus den Strukturelementen -Phe-, -Cyc-, -Phe-Phe-, -Phe-Cyc-, -Cyc-Cyc-, -Pyr-, -Dio-, -Py-, -G-Phe-, -G-Cyc- und deren Spiegelbildern gebildeten Gruppe, wobei Phe unsubstituiertes oder durch Fluor substituiertes 1,4-Phenylen, Cyc trans-1,4-Cyclohexylen, Pyr Pyrimidin-2,5-diyl oder Pyridin-2,5-diyl, Dio 1,3-Dioxan-2,5-diyl, Py Tetrahydropyran-2,5-diyl- und G 2-(trans-1,4-Cyclohexyl)-ethyl bedeuten. Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe, Py und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS oder -(O)ᵢCH₃₋ₖFₖ, wobei i 0 oder 1 und k 1, 2 oder 3 ist.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im Folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋ₖFₖ, wobei i 0 oder 1 und k 1, 2 oder 3 ist. Die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a bis 5a angegebenen Bedeutungen und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN. Diese Untergruppe wird im Folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a bis 5a angegebenen Bedeutungen und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:

| | |
|---|---|
| Gruppe A: | 0 bis 90 %, vorzugsweise 20 bis 90 %, besonders bevorzugt 30 bis 90 %; |
| Gruppe B: | 0 bis 80 %, vorzugsweise 10 bis 80 %, besonders bevorzugt 10 bis 65 %; |
| Gruppe C: | 0 bis 80 %, vorzugsweise 0 bis 80 %, besonders bevorzugt 0 bis 50 %; |

wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 3 bis 30 %, der erfindungsgemäßen Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe, chirale Dotierstoffe, Stabilisatoren oder Nanopartikel zugesetzt werden. Die einzelnen zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben. Die erfindungsgemäßen Flüssigkristallmischungen ermöglichen eine bedeutende Erweiterung des zur Verfügung stehenden Parameterraumes.

Gegenstand der Erfindung sind auch elektrooptische Anzeigen (insbesondere TFT-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Der Ausdruck "Alkyl" umfasst unverzweigte und verzweigte Alkylgruppen mit 1-15 Kohlenstoffatomen, insbesondere die unverzweigten Gruppen Methyl, Ethyl, n-Propyl, n-Butyl, n-Pentyl, n-Hexyl und n-Heptyl. Gruppen mit 2-5 Kohlenstoffatomen sind im allgemeinen bevorzugt.

Der Ausdruck "Alkenyl" umfasst unverzweigte und verzweigte Alkenylgruppen mit bis zu 15 Kohlenstoffatomen, insbesondere die unverzweigten Gruppen. Besonders bevorzugte Alkenylgruppen sind C₂₋C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃. Der Ausdruck "halogenierter Alkenylrest" und verwandte Ausdrücke erklären sich entsprechend.

Die Gesamtmenge an Verbindungen der Formeln I in den erfindungsgemäßen Gemischen ist nicht kritisch. Die Gemische können daher eine oder mehrere weitere Komponenten enthalten zwecks Optimierung verschiedener Eigenschaften.

Der Aufbau der erfindungsgemäßen Matrix-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der Matrix-Anzeige, insbesondere auch Matrix-Anzeigeelemente auf Basis von poly-Si TFT.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die folgenden Beispiele erläutern die Erfindung, ohne sie begrenzen zu sollen. Der Fachmann wird in der Lage sein, den Beispielen Details zur Durchführung zu entnehmen, die in der allgemeinen Beschreibung nicht im Einzelnen aufgeführt sind, sie nach allgemeinen Fachkenntnissen zu verallgemeinern und auf eine spezielle Problemstellung anzuwenden.

Vor- und nachstehend bedeuten Prozentangaben Gewichtsprozent. Alle Temperaturen sind in Grad Celsius angegeben. Ferner bedeuten K = kristalliner Zustand, N = nematische Phase, Sm = smektische Phase (spezieller SmA, SmB, etc.), Tg = Glaspunkt und I = isotrope Phase. Die Angaben zwischen diesen Symbolen stellen die Übergangstemperaturen dar. Δn bedeutet optische Anisotropie (589 nm, 20 °C), Δε die dielektrische Anisotropie (1kHz, 20 °C) und γ₁ die Rotationsviskosität (20°C; in der Einheit mPa·s).

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal^{®} - Physical Properties of Liquid Crystals - Description of the Measurements Methods", 1998, Merck KGaA, Darmstadt. Die dielektrische Anisotropie Δε der einzelnen Substanzen wird bei 20 °C und 1 kHz bestimmt. Dazu werden 5-10 Gew.% der zu untersuchenden Substanz in der dielektrisch positiven Mischung ZLI-4792 (Merck KGaA) gelöst gemessen und der Messwert auf eine Konzentration von 100 % extrapoliert. Die optische Anisotropie Δn wird bei 20°C und einer Wellenlänge von 589,3 nm bestimmt, die Rotationsviskosität γ₁ bei 20°C, beide ebenfalls durch lineare Extrapolation.

In der vorliegenden Anmeldung, bedeutet, wenn nicht ausdrücklich anders angegeben, die Pluralform eines Begriffs sowohl die Singularform als auch die Pluralform, und umgekehrt. Weitere Kombinationen der Ausführungsformen und Varianten der Erfindung gemäß der Beschreibung ergeben sich auch aus den angefügten Ansprüchen oder aus Kombinationen von mehreren dieser Ansprüche.

Folgende Abkürzungen werden verwendet:

| | |
|---|---|
| RT | Raumtemperatur |
| THF | Tetrahydrofuran |
| MTB-Ether | Methyl-tert-butylether |
| ges. | gesättigt |
| dest. | destilliert |

### Beispiele

Die vorliegende Erfindung wird durch die nachfolgenden nicht einschränkenden Beispiele detailliert beschrieben.

### Beispiel 1: 2-[4-[3-Fluor-4-(3,4,5-trifluorphenyl)phenyl]cyclohex-3-en-1-yl]-5-propyl-1,3-dioxan

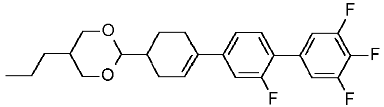

### Stufe 1.1: 4-(5-Propyl-1,3-dioxan-2-yl)cyclohexanon

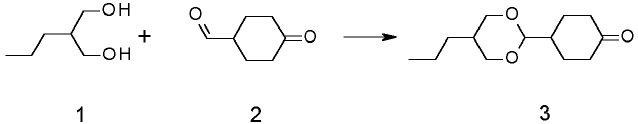

Eine Lösung von 2-Propylpropan-1,3-diol **1** (23,9 g, 193 mmol) und 4-Oxocyclohexancarbaldehyd **2** (CAS 96184-81-5, 30,0 g, 170 mmol) in Dichlormethan (270 ml) wird mit Toluol-4-sulfonsäure-Monohydrat (6,4 g, 32 mmol) versetzt und am Wasserabscheider refluxiert. Nach 90 min wird das Reaktionsgemisch auf RT abgekühlt und über Kieselgel chromatografiert (Dichlormethan /Ethylacetat 9:1). 4-(5-Propyl-1,3-dioxan-2-yl)cyclohexanon **3** wird als gelbes, klares Öl isoliert, welches zu einer Kristallmasse erstarrt.

### Stufe 1.2: 1-[3-Fluor-4-(3,4,5-trifluorphenyl)phenyl]-4-(5-propyl-1,3-dioxan-2-yl)cyclohexanol

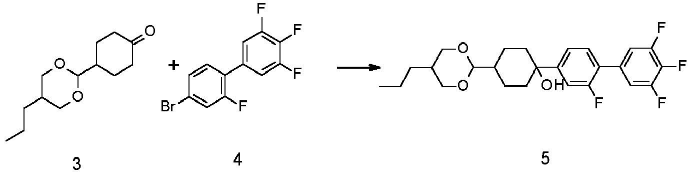

Eine Lösung von Isopropylmagnesiumchlorid-Lithiumchlorid (116 ml, 150 mmol, 1,3 mol/l in THF) wird vorgelegt und bei 30°C eine Lösung aus 5-(4-Brom-2-fluor-phenyl)-1,2,3-trifluor-benzol **4** (CAS 187804-77-9, 25,0 g, 80 mmol) in THF (150 ml) zugetropft. Nach 60 min wird eine Lösung von 4-(5-Propyl-1,3-dioxan-2-yl)cyclohexanon **3** (25,0 g, 80 mmol) in THF (150 ml) bei maximal 30°C zugetropft. Nach weiteren 60 min wird das Reaktionsgemisch mit dest. Wasser versetzt und mit Salzsäure (1 M) auf pH = 5 eingestellt. Die wässrige Phase wird abgetrennt und mit MTB-Ether extrahiert. Die vereinigten organischen Phasen werden mit ges. Natriumhydrogencarbonat-Lösung gewaschen, über Natriumsulfat getrocknet, filtriert und im Vakuum eingeengt. Der Rückstand ergibt 1-[3-Fluor-4-(3,4,5-trifluorphenyl)phenyl]-4-(5-propyl-1,3-dioxan-2-yl)cyclohexanol **5** als gelbliche Kristalle.

### Stufe 1.3: 2-[4-[3-Fluor-4-(3,4,5-trifluorphenyl)phenyl]cyclohex-3-en-1-yl]-5-propyl-1,3-dioxan

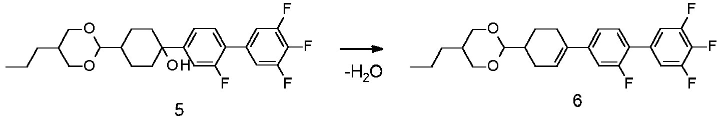

Eine Lösung von 1-[3-Fluor-4-(3,4,5-trifluorphenyl)phenyl]-4-(5-propyl-1,3-dioxan-2-yl)cyclohexanol **5** (15,8 g, 15 mmol) in Toluol (80 ml) wird mit Toluol-4-sulfonsäure-Monohydrat (0,5 g, 3 mmol) versetzt und am Wasserabscheider für 3 h unter Rückfluss erhitzt. Das Reaktionsgemisch wird auf RT abgekühlt, mit Heptan verdünnt und über Kieselgel chromatografiert (Heptan/MTB-Ether 95:5). Nach Kristallisation aus 2-Propanol und Heptan wird 2-[4-[3-Fluor-4-(3,4,5-trifluorphenyl)-phenyl]cyclohex-3-en-1-yl]-5-propyl-1,3-dioxan **6** als farbloser Feststoff isoliert. Die Verbindung **6** zeigt folgendes Phasenverhalten:
K 74 SmA 168 N 191 I.
Δε = 30
Δn = 0,18

### Beispiel 2: 2-[4-[3-Fluor-4-(3,4,5-trifluorphenyl)phenyl]cyclohex-3-en-1-yl]-5-butyl-1,3-dioxan

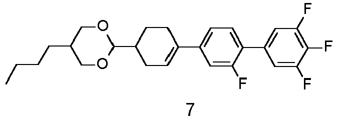

Analog zu 2-[4-[3-Fluor-4-(3,4,5-trifluorphenyl)phenyl]cyclohex-3-en-1-yl]-5-propyl-1,3-dioxan **6** wird 2-[4-[3-Fluor-4-(3,4,5-trifluorphenyl)phenyl]-cyclohex-3-en-1-yl]-5-butyl-1,3-dioxan **7** synthetisiert. Die Verbindung **7** zeigt folgendes Phasenverhalten:
K64 SmA 174 N 190 I.
Δε = 28
Δn = 0,18

### Beispiel 3: 2-[4-[4-[3,5-Difluor-4-(trifluormethyl)phenyl]-3-fluorphenyl]cyclohex-3-en-1-yl]-5-propyl-1,3-dioxan

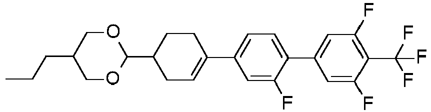

### Stufe 3.1: [3,5-Difluor-4-(trifluormethyl)phenyl]-boronsäure

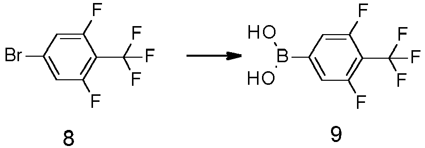

Eine Lösung von 5-Brom-1,3-difluor-2-(trifluormethyl)benzol **8** (CAS 156243-64-0, 200 g, 760 mmol) in THF (800 ml) wird auf -5°C eingekühlt und mit Isopropylmagnesiumchlorid (420 ml, 840 mmol, 2,0 mol/l in THF) versetzt. Nach 1 h wird eine Lösung von Trimethylborat (106 ml, 950 mmol) in THF (100 ml) bei -5°C zugegeben und das Reaktionsgemisch über Nacht bei RT gerührt. Das Reaktionsgemisch wird auf eiskalte Salzsäure (410 ml, 2 M) gegeben und mit MTB-Ether extrahiert. Die Phasen werden getrennt, die organische Phase wird mit dest. Wasser gewaschen, über Natriumsulfat getrocknet, filtriert und im Vakuum eingeengt. Das Rohprodukt wird durch Kristallisation aus Heptan gereinigt und ergibt [3,5-Difluor-4-(trifluormethyl)phenyl]-boronsäure **9** als beigen Feststoff.

### Stufe 3.2: 5-(4-Brom-2-fluor-phenyl)-1,3-difluor-2-(trifluormethyl)benzol

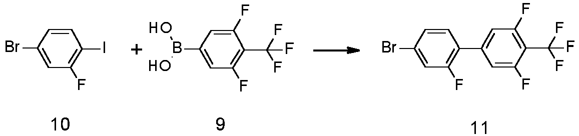

Eine Lösung von Natriummetaborat-Tetrahydrat (8,9 g, 64 mmol) in dest. Wasser (23 ml) wird vorgelegt, THF (10 ml), Bis(triphenylphosphin)-palladium(II)-chlorid (15,2% Pd) (0,6 g, 0,9 mmol) und Hydraziniumhydroxid (0,04 ml, 0,9 mmol) zugegeben und 5 min gerührt. Dann werden 4-Brom-2-fluor-1-iod-benzol **10** (CAS 105931-73-5, 12,7 g, 42 mmol), [3,5-Difluor-4-(trifluormethyl)phenyl]-boronsäure **9** (10,0 g, 42 mmol) und THF (40 ml) zugegeben und das Reaktionsgemisch über Nacht refluxiert. Das Reaktionsgemisch wird mit dest. Wasser versetzt und mit MTB-Ether verdünnt. Die wässrige Phase wird abgetrennt und mit MTB-Ether extrahiert. Die vereinigten organischen Phasen werden mit ges. Natriumchlorid-Lösung gewaschen, über Natriumsulfat getrocknet, filtriert und im Vakuum eingeengt. Das Rohprodukt wird über Kieselgel chromatografiert (Heptan) und ergibt 5-(4-Brom-2-fluor-phenyl)-1,3-difluor-2-(trifluormethyl)benzol **11** als klares, farbloses Öl.

### Stufe 3.3: 1-[4-[3,5-Difluor-4-(trifluormethyl)phenyl]-3-fluor-phenyl]-4-(5-propyl-1,3-dioxan-2-yl)cyclohexanol

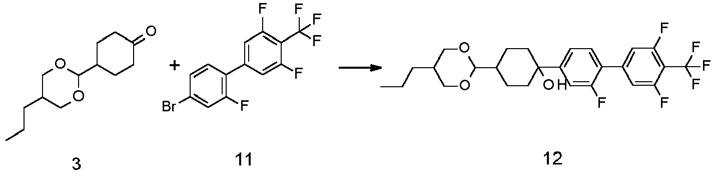

Isopropylmagnesiumchlorid (15,7 ml, 31,4 mmol, 2 mol/l in THF) wird vorgelegt und bei 30°C eine Lösung aus 5-(4-Brom-2-fluor-phenyl)-1,3-difluor-2-(trifluormethyl)benzol **11** (5,8 g, 15,7 mmol) in THF (50 ml) zugetropft. Nach 60 min wird eine Lösung von 4-(5-Propyl-1,3-dioxan-2-yl)cyclohexanon **3** (5,0 g, 15,7 mmol) in THF (15 ml) bei maximal 20°C zugetropft. Nach weiteren 60 min wird das Reaktionsgemisch mit dest. Wasser versetzt und mit Salzsäure (1 M) auf pH = 5 eingestellt. Die wässrige Phase wird abgetrennt und mit MTB-Ether extrahiert. Die vereinigten organischen Phasen werden mit ges. Natriumhydrogencarbonat-Lösung gewaschen, über Natriumsulfat getrocknet, filtriert und im Vakuum eingeengt. Der Rückstand ergibt 1-[4-[3,5-Difluor-4-(trifluormethyl)phenyl]-3-fluor-phenyl]-4-(5-propyl-1,3-dioxan-2-yl)cyclohexanol **12** als bräunliche Kristallmasse.

### Stufe 3.4: 2-[4-[4-[3,5-Difluor-4-(trifluormethyl)phenyl]-3-fluorphenyl]cyclohex-3-en-1-yl]-5-propyl-1,3-dioxan

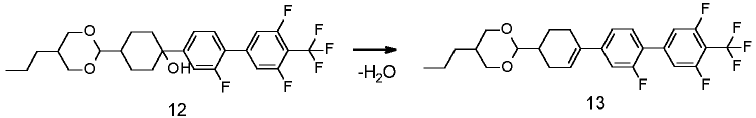

Eine Lösung von 1-[4-[3,5-Difluor-4-(trifluormethyl)phenyl]-3-fluor-phenyl]-4-(5-propyl-1,3-dioxan-2-yl)cyclohexanol **12** (10,1 g, 8,2 mmol) in Toluol (45 ml) wird mit Toluol-4-sulfonsäure-Monohydrat (0,3 g, 1,6 mmol) versetzt und am Wasserabscheider für 2 h refluxiert. Das Reaktionsgemisch wird auf RT abgekühlt, im Vakuum eingeengt und über Kieselgel chromatografiert (Heptan/MTB-Ether 95:5). Nach Kristallisation aus 2-Propanol und n-Heptan wird 2-[4-[4-[3,5-Difluor-4-(trifluormethyl)phenyl]-3-fluor-phenyl]cyclohex-3-en-1-yl]-5-propyl-1,3-dioxan **13** als farbloser Feststoff isoliert. Die Verbindung **13** zeigt folgendes Phasenverhalten:
K 124 SmA173 N 186 I.
Δε = 38
Δn = 0,19

### Beispiel 4: 2-[4-[4-[3,5-Difluor-4-(trifluormethoxy)phenyl]-3-fluorphenyl]cyclohex-3-en-1-yl]-5-propyl-1,3-dioxan

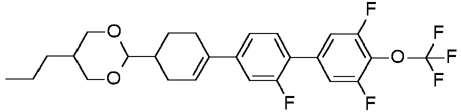

### Stufe 4.1: 2-[3,5-Difluor-4-(trifluormethoxy)phenyl]-4,4,5,5-tetramethyl-1,3-dioxa-2-borolane

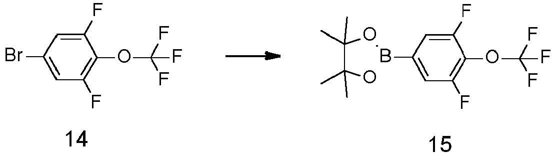

Zu einer Lösung von 5-Brom-1,3-difluor-2-(trifluormethoxy)benzol **14** (CAS 115467-07-7, 50,0 g, 180 mmol) in 1,4-Dioxan (430 ml) werden Kaliumacetat (53,2 g, 540 mmol), 1,1'-Bis(biphenylphosphin)ferrocenpalladiumdichlorid (4,0 g, 5,4 mmol) und Bis-(pinacolato)-dibor (70,2 g, 271 mmol) zugegeben und über Nacht refluxiert. Das Reaktionsgemisch wird auf RT abgekühlt, mit dest. Wasser versetzt und mit MTB-Ether verdünnt. Die Phasen werden getrennt, die wässrige Phase wird mit MTB-Ether extrahiert, die vereinigten organischen Phasen werden mit ges. Natriumchlorid-Lösung gewaschen, über Natriumsulfat getrocknet, filtriert und im Vakuum eingeengt. Das Rohprodukt wird über Kieselgel chromatografiert (Toluol) und aus Ethanol kristallisiert und ergibt 2-[3,5-Difluor-4-(trifluormethoxy)phenyl]-4,4,5,5-tetramethyl-1,3-dioxa-2-borolan **15** als farblosen Feststoff.

### Stufe 4.2: 5-(4-Brom-2-fluor-phenyl)-1,3-difluor-2-(trifluormethoxy)benzol

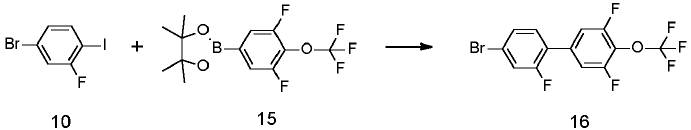

Eine Lösung von Natriummetaborat-Tetrahydrat (10,2 g, 74 mmol) in dest. Wasser (33,2 ml) wird vorgelegt, THF (10 ml), Bis(triphenylphosphin)-palladium(II)-chlorid (15,2% Pd) (1,7 g, 2,5 mmol) und Hydraziniumhydroxid (0,12 ml, 2,5 mmol) zugegeben und 5 min gerührt. Dann werden 4-Brom-2-fluor-1-iod-benzol **10** (18,5 g, 61 mmol), 2-[3,5-Difluor-4-(trifluormethoxy)phenyl]-4,4,5,5-tetramethyl-1,3-dioxa-2-borolan **15** (20,0 g, 61 mmol) und THF (65 ml) zugegeben und das Reaktionsgemisch über Nacht refluxiert. Das Reaktionsgemisch wird mit dest. Wasser versetzt und mit MTB-Ether verdünnt. Die wässrige Phase wird abgetrennt und mit MTB-Ether extrahiert. Die vereinigten organischen Phasen werden mit ges. Natriumchlorid-Lösung gewaschen, über Natriumsulfat getrocknet, filtriert und im Vakuum eingeengt. Das Rohprodukt wird über Kieselgel chromatografiert (Heptan) und in Heptan umkristallisiert und ergibt 5-(4-Brom-2-fluor-phenyl)-1,3-difluor-2-(trifluormethoxy)benzol **16** als farblosen Feststoff.

### Stufe 4.3: 1-[4-[3,5-Difluor-4-(trifluormethoxy)phenyl]-3-fluor-phenyl]-4-(5-propyl-1,3-dioxan-2-yl)cyclohexanol

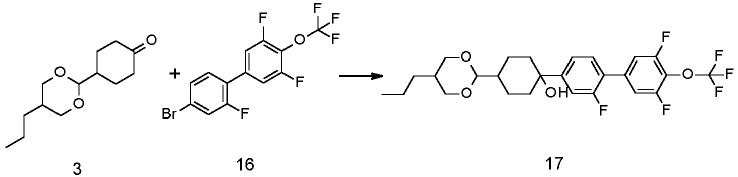

Isopropylmagnesiumchlorid-Lithiumchlorid (23,7 ml, 30,8 mmol, 1,3 mol/l in THF) wird vorgelegt und bei 30°C eine Lösung aus 5-(4-Brom-2-fluorphenyl)-1,3-difluor-2-(trifluormethoxy)benzol **16** (5,5 g, 14,6 mmol) in THF (25 ml) zugetropft. Nach 60 min wird eine Lösung von 4-(5-Propyl-1,3-dioxan-2-yl)cyclohexanon **3** (5,0 g, 15,4 mmol) in THF (37 ml) bei maximal 20°C zugetropft und über Nacht gerührt. Dann wird das Reaktionsgemisch mit dest. Wasser versetzt und mit Salzsäure (1 M) auf pH = 5 eingestellt. Die wässrige Phase wird abgetrennt und mit MTB-Ether extrahiert. Die vereinigten organischen Phasen werden mit ges. Natriumhydrogencarbonat-Lösung gewaschen, über Natriumsulfat getrocknet, filtriert und im Vakuum eingeengt. Der Rückstand ergibt 1-[4-[3,5-Difluor-4-(trifluormethoxy)phenyl]-3-fluor-phenyl]-4-(5-propyl-1,3-dioxan-2-yl)cyclohexanol **17** als gelbe Kristallmasse.

### Stufe 4.4: 2-[4-[4-[3,5-Difluor-4-(trifluormethoxy)phenyl]-3-fluorphenyl]cyclohex-3-en-1-yl]-5-propyl-1,3-dioxan

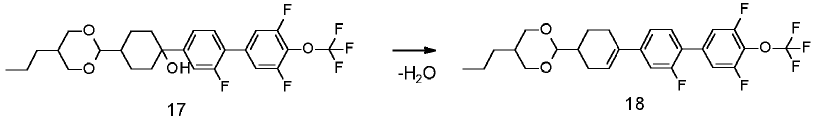

Eine Lösung von 1-[4-[3,5-Difluor-4-(trifluormethoxy)phenyl]-3-fluorphenyl]-4-(5-propyl-1,3-dioxan-2-yl)cyclohexanol **17** (10,8 g, 9,9 mmol) in Toluol (53 ml) wird mit Toluol-4-sulfonsäure-Monohydrat (0,3 g, 2,0 mmol) versetzt und am Wasserabscheider für 2 h refluxiert. Das Reaktionsgemisch wird auf RT abgekühlt, im Vakuum eingeengt und über Kieselgel chromatografiert (Heptan/MTB-Ether 95:5). Nach Kristallisation aus 2-Propanol und Heptan wird 2-[4-[4-[3,5-Difluor-4-(trifluormethoxy)phenyl]-3-fluor-phenyl]cyclohex-3-en-1-yl]-5-propyl-1,3-dioxan **18** als farbloser Feststoff isoliert. Die Verbindung **18** zeigt folgendes Phasenverhalten:
K95 Sm 213 I.
Δε = 30
Δn = 0,18

### Beispiel 5: 2-[4-[3-Fluor-4-(3,4,5-trifluorphenyl)phenyl]cyclohex-3-en-1-yl]-5-propyl-tetrahydropyran

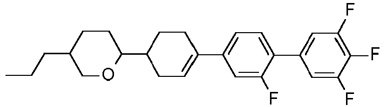

### Stufe 5.1: 4-(5-Propyltetrahydropyran-2-yl)cyclohexanon

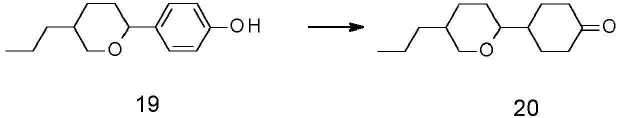

4-(5-Propyltetrahydropyran-2-yl)phenol **19** (CAS 879544-24-8, 50,0 g, 227 mmol) wird in Xylol (330 ml) gelöst, mit Pd/C (5%, 4,4 g) und Natriumcarbonat (0,2 g, 2,3 mmol) versetzt und unter Druck mit Wasserstoff (10,2 I) hydriert. Nach 8 h wird die Lösung filtriert und im Vakuum eingeengt. Das Rohprodukt wird in n-Heptan (200 ml) aufgenommen, mit einer Mischung aus Schwefeltrioxid-Pyridin-Komplex (37,0 g, 233 mmol) und Celite (50 g) versetzt und über Nacht bei RT gerührt. Danach werden erneut Celite (20 g) und Kieselgel (20 g) nachgegeben und gerührt. Nach 90 min wird das Reaktionsgemisch abgesaugt. Das Filtrat wird mit dest. Wasser und ges. Natriumhydrogencarbonat-Lösung gewaschen, über Natriumsulfat getrocknet, filtriert und im Vakuum eingeengt. Der Rückstand ergibt 4-(5-Propyltetrahydropyran-2-yl)cyclohexanon **20** als klares, gelbliches Öl.

### Stufe 5.2: 1-[3-Fluor-4-(3,4,5-trifluorphenyl)phenyl]-4-(5-propyltetrahydropyran-2-yl)cyclohexanol

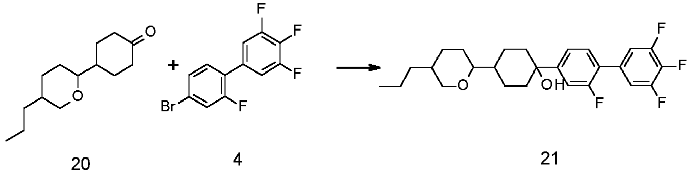

Eine Lösung von Isopropylmagnesiumchlorid (22,9 ml, 46 mmol, 2,0 mol/l in THF) wird vorgelegt und bei 30°C eine Lösung von 5-(4-Brom-2-fluorphenyl)-1,2,3-trifluor-benzol **4** (7,0 g, 23 mmol) in THF (50 ml) zugetropft. Nach 60 min wird eine Lösung von 4-(5-Propyltetrahydropyran-2-yl)cyclohexanon **20** (5,1 g, 23 mmol) in THF (50 ml) bei maximal 30°C zugetropft. Nach weiteren 60 min wird das Reaktionsgemisch mit dest. Wasser versetzt und mit Salzsäure (1 M) auf pH = 5 eingestellt. Die wässrige Phase wird abgetrennt und mit MTB-Ether extrahiert. Die vereinigten organischen Phasen werden mit ges. Natriumhydrogencarbonat-Lösung gewaschen, über Natriumsulfat getrocknet, filtriert und im Vakuum eingeengt. Der Rückstand ergibt 1-[3-Fluor-4-(3,4,5-trifluorphenyl)phenyl]-4-(5-propyltetrahydropyran-2-yl)cyclohexanol **21** als gelbliche Kristalle.

### Stufe 5.3: 2-[4-[3-Fluor-4-(3,4,5-trifluorphenyl)phenyl]cyclohex-3-en-1-yl]-5-propyl-tetrahydropyran

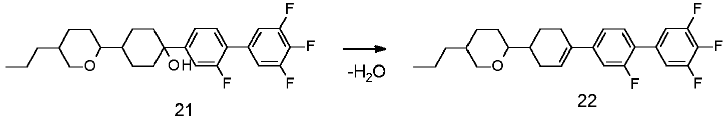

Eine Lösung von 1-[3-Fluor-4-(3,4,5-trifluorphenyl)phenyl]-4-(5-propyltetrahydropyran-2-yl)cyclohexanol **21** (12,2 g, 15 mmol) in Toluol (80 ml) wird mit Toluol-4-sulfonsäure-Monohydrat (0,5 g, 3 mmol) versetzt und am Wasserabscheider für 90 min refluxiert. Das Reaktionsgemisch wird auf RT abgekühlt, mit n-Heptan verdünnt und über Kieselgel chromatografiert (Heptan/MTB-Ether 95:5). Nach Kristallisation aus 2-Propanol und n-Heptan wird 2-[4-[3-Fluor-4-(3,4,5-trifluorphenyl)phenyl]-cyclohex-3-en-1-yl]-5-propyl-tetrahydropyran **22** in Form von farblosen Kristallen isoliert. Verbindung **22** zeigt folgendes Phasenverhalten:
K 95 N 191 I.
Δε = 23
Δn = 0,17

### Beispiel 6: 2-[4-[4-[3,5-Difluor-4-(trifluormethyl)phenyl]-3-fluorphenyl]cyclohex-3-en-1-yl]-5-propyl-tetrahydropyran

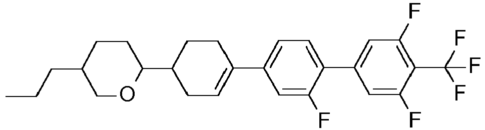

### Stufe 6.1: 1-[4-[3,5-Difluor-4-(trifluormethyl)phenyl]-3-fluor-phenyl]-4-(5-propyltetrahydropyran-2-yl)cyclohexanol

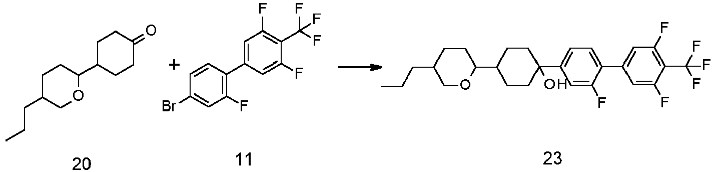

Isopropylmagnesiumchlorid (10,8 ml, 22 mmol, 2 mol/l in THF) wird vorgelegt und bei 30°C eine Lösung aus 5-(4-Brom-2-fluor-phenyl)-1,3-difluor-2-(trifluormethyl)benzol **11** (4,0 g, 11 mmol) in THF (20 ml) zugetropft. Nach 60 min wird eine Lösung von 4-(5-Propyltetrahydropyran-2-yl)cyclohexanon **20** (2,5 g, 11 mmol) in THF (15 ml) bei maximal 20°C zugetropft. Nach weiteren 60 min wird das Reaktionsgemisch mit dest. Wasser versetzt und mit Salzsäure (1 M) auf pH = 5 eingestellt. Die wässrige Phase wird abgetrennt und mit MTB-Ether extrahiert. Die vereinigten organischen Phasen werden mit ges. Natriumhydrogencarbonat-Lösung gewaschen, über Natriumsulfat getrocknet, filtriert und im Vakuum eingeengt. Der Rückstand ergibt 1-[4-[3,5-Difluor-4-(trifluormethyl)phenyl]-3-fluor-phenyl]-4-(5-propyltetrahydropyran-2-yl)cyclohexanol **23** als gelbliche Kristallmasse.

### Stufe 6.2: 2-[4-[4-[3,5-Difluor-4-(trifluormethyl)phenyl]-3-fluorphenyl]cyclohex-3-en-1-yl]-5-propyl-tetrahydropyran

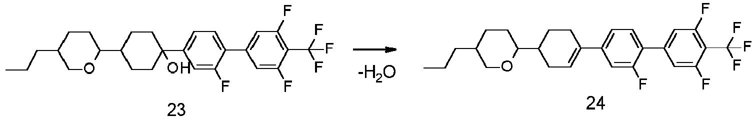

Eine Lösung von 1-[4-[3,5-Difluor-4-(trifluormethyl)phenyl]-3-fluor-phenyl]-4-(5-propyltetrahydropyran-2-yl)cyclohexanol **23** (7,1 g, 7 mmol) in Toluol (40 ml) wird mit Toluol-4-sulfonsäure-Monohydrat (0,3 g, 1,5 mmol) versetzt und am Wasserabscheider für 2 h refluxiert. Das Reaktionsgemisch wird auf RT abgekühlt, im Vakuum eingeengt und über Kieselgel chromatografiert (Heptan/MTB-Ether 95:5). Nach Kristallisation aus 2-Propanol und *n*-Heptan wird 2-[4-[4-[3,5-Difluor-4-(trifluormethyl)phenyl]-3-fluor-phenyl]cyclohex-3-en-1-yl]-5-propyl-tetrahydropyran **24** in Form von farblosen Kristallen isoliert. Verbindung **24** zeigt folgendes Phasenverhalten:
K 98 N 182 I.
Δε = 31
Δn = 0,19

### Beispiel 7: 2-[4-[4-[3,5-Difluor-4-(trifluormethoxy)phenyl]-3-fluorphenyl]cyclohex-3-en-1-yl]-5-propyl-tetrahydropyran

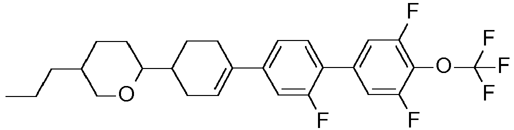

### Stufe 7.1: 1-[4-[3,5-Difluor-4-(trifluormethoxy)phenyl]-3-fluor-phenyl]-4-(5-propyltetrahydropyran-2-yl)cyclohexanol

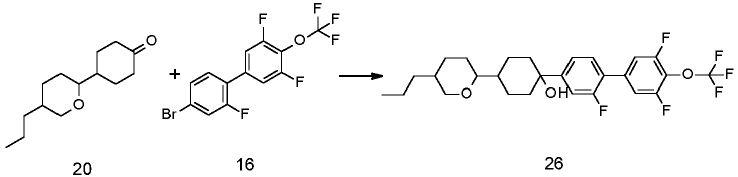

Isopropylmagnesiumchlorid (13,1 ml, 26 mmol, 2,0 mol/l in THF) wird vorgelegt und bei 30°C eine Lösung aus 5-(4-Brom-2-fluor-phenyl)-1,3-difluor-2-(trifluormethoxy)benzol **16** (5,0 g, 13 mmol) in THF (25 ml) zugetropft. Nach 60 min wird eine Lösung von 4-(5-Propyltetrahydropyran-2-yl)cyclohexanon **20** (3,0 g, 13 mmol) in THF (37 ml) bei maximal 20°C zugetropft und über Nacht gerührt. Dann wird das Reaktionsgemisch mit dest. Wasser versetzt und mit Salzsäure (1 M) auf pH = 5 eingestellt. Die wässrige Phase wird abgetrennt und mit MTB-Ether extrahiert. Die vereinigten organischen Phasen werden mit ges. Natriumhydrogencarbonat-Lösung gewaschen, über Natriumsulfat getrocknet, filtriert und im Vakuum eingeengt. Der Rückstand ergibt 1-[4-[3,5-Difluor-4-(trifluormethoxy)phenyl]-3-fluor-phenyl]-4-(5-propyltetrahydropyran-2-yl)cyclohexanol **26** als gelbliches Öl.

### Stufe 7.2: 2-[4-[4-[3,5-Difluor-4-(trifluormethoxy)phenyl]-3-fluorphenyl]cyclohex-3-en-1-yl]-5-propyl-tetrahydropyran

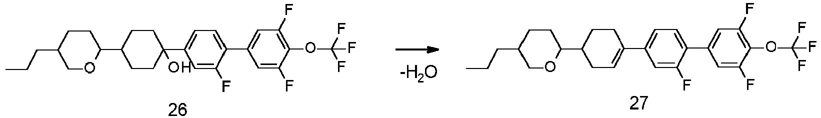

Eine Lösung von 1-[4-[3,5-Difluor-4-(trifluormethoxy)phenyl]-3-fluorphenyl]-4-(5-propyltetrahydropyran-2-yl)cyclohexanol **26** (8,7 g, 8 mmol) in Toluol (40 ml) wird mit Toluol-4-sulfonsäure-Monohydrat (0,3 g, 2 mmol) versetzt und am Wasserabscheider für 2 h refluxiert. Das Reaktionsgemisch wird auf RT abgekühlt, im Vakuum eingeengt und über Kieselgel chromatografiert (Heptan/MTB-Ether 95:5). Nach Kristallisation aus 2-Propanol und *n*-Heptan wird 2-[4-[4-[3,5-Difluor-4-(trifluormethoxy)phenyl]-3-fluor-phenyl]cyclohex-3-en-1-yl]-5-propyl-tetrahydropyran **27** als farbloser Feststoff isoliert. Verbindung **27** zeigt folgendes Phasenverhalten:
K78 Sm 117 N 206 I
Δε = 24
Δn = 0,18

### Analog werden hergestellt:

### Beispiel 8: 2-[4-[3-Fluor-4-(2-methyl-3,4,5-trifluorphenyl)phenyl]cyclohex-3-en-1-yl]-5-propyl-1,3-dioxan

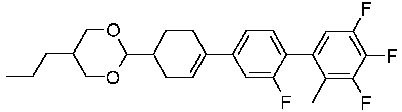

### Beispiel 9: 2-[4-[3-Fluor-4-(2-methyl-3,4,5-trifluorphenyl)phenyl]cyclohex-3-en-1-yl]-5-propyl-tetrahydropyran

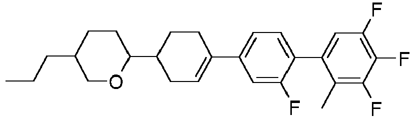

## Patentansprüche

1. Verbindungen der Formel I, 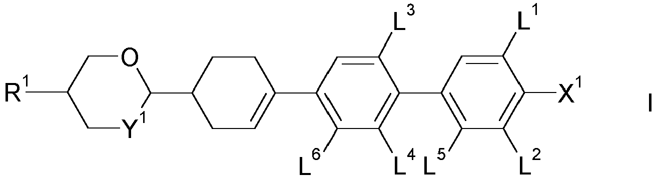 worin
X¹ F, CF₃, OCF₃, OCHF₂, CHF₂, SCN oder CN,
Y¹ O oder CH₂,
R¹ Alkyl oder Alkenyl mit bis zu 8 Kohlenstoffatomen,
L¹ und L² unabhängig voneinander H oder F,
L³ H oder F,
L⁴ H oder F, und
L⁵ und L⁶ unabhängig voneinander H oder CH₃, bedeuten.

2. Verbindungen nach Anspruch 1, **dadurch gekennzeichnet, dass**
L⁴ H
bedeutet.

3. Verbindungen nach Anspruch 1 oder 2, **dadurch gekennzeichnet, dass**
X¹ F, OCF₃ oder CF₃ bedeutet.

4. Verbindungen nach einem oder mehreren der Ansprüche 1 bis 3, **dadurch gekennzeichnet, dass** L¹ und L² F bedeuten.

5. Verbindungen nach einem oder mehreren der Ansprüche 1 bis 4, **dadurch gekennzeichnet, dass** Y¹ O bedeutet.

6. Verbindungen nach einem oder mehreren der Ansprüche 1 bis 4, **dadurch gekennzeichnet, dass** Y¹ CH₂ bedeutet.

7. Verbindungen nach einem oder mehreren der Ansprüche 1 bis 6 ausgewählt aus den folgenden Formeln: 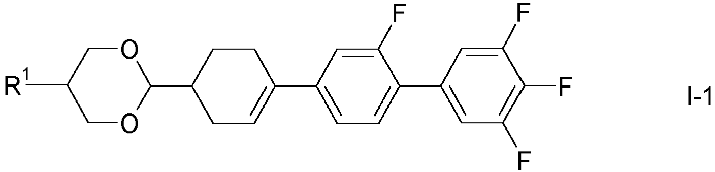 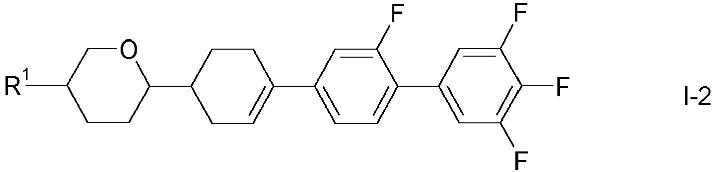 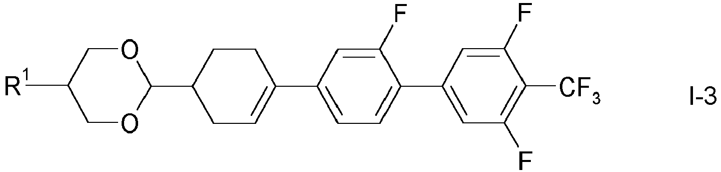 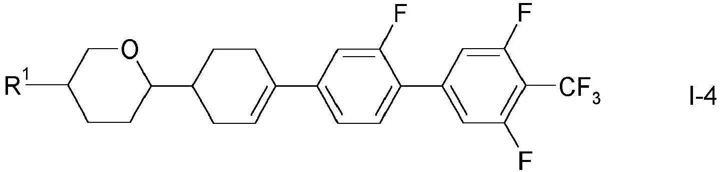 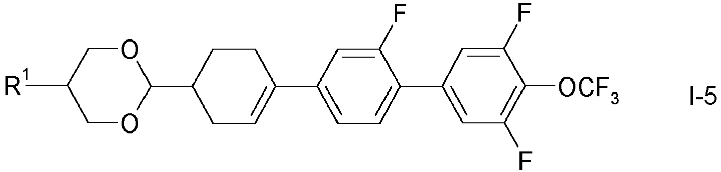 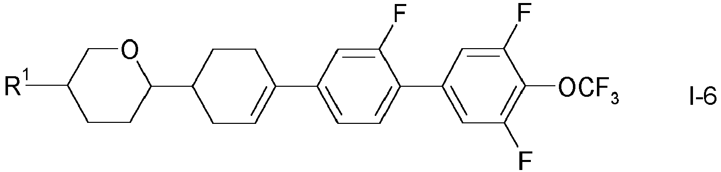 worin R¹ die in Anspruch 1 angegebene Bedeutung hat.

8. Verbindungen nach einem oder mehreren der Ansprüche 1 bis 7, **dadurch gekennzeichnet, dass** R¹ einen geradkettigen Alkylrest mit 1 bis 7 C-Atomen oder einen unverzweigten Alkenylrest mit 2 bis 8 C-Atomen bedeutet.

9. Verfahren zur Herstellung von Verbindungen der Formel I nach einem oder mehreren der Ansprüche 1 bis 8 umfassend einen Verfahrensschritt, in dem eine Arylhalogenverbindung der Formel II 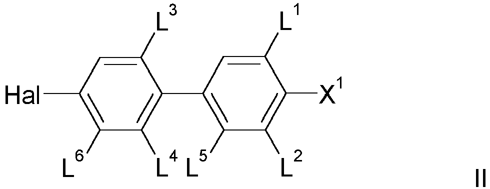 worin X¹, L¹, L², L³ und L⁴, wie in Anspruch 1 definiert sind, und
Hal Br, I oder C!, bedeutet,
mit einer Verbindung der Formel III 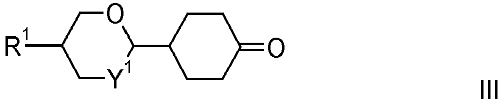
worin
R¹ und Y¹ wie in Anspruch 1 definiert sind,
zu einer Verbindung der Formel IV umgesetzt wird, 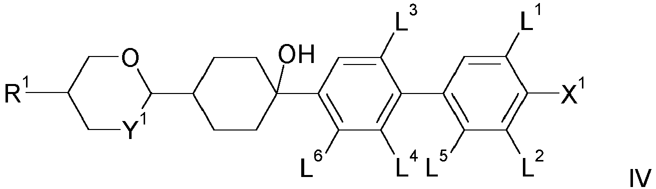
und weiter zu einer Verbindung der Formel I 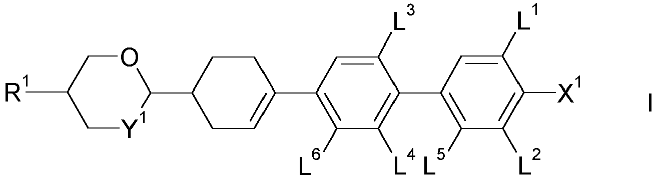
worin die Gruppen R¹, Y¹, L¹, L², L³, L⁴ und X¹ wie in Anspruch 1 definiert sind,
umgesetzt wird.

10. Verwendung einer oder mehrerer Verbindungen der Formel I nach einem oder mehreren der Ansprüche 1 bis 8 als Komponente in einem flüssigkristallinen Medium.

11. Flüssigkristallines Medium enthaltend mindestens zwei mesogene Verbindungen, **dadurch gekennzeichnet, dass** es mindestens eine Verbindung der Formel I nach einem oder mehreren der Ansprüche 1 bis 8 enthält.

12. Verwendung des flüssigkristallinen Mediums nach Anspruch 11 für elektrooptische Zwecke.

13. Elektrooptische Flüssigkristallanzeige enthaltend ein flüssigkristallines Medium nach Anspruch 11.

## Claims

1. Compounds of the formula I, 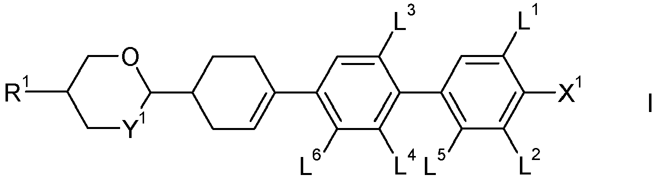 in which
X¹ denotes F, CF₃, OCF₃, OCHF₂, CHF₂, SCN or CN,
Y¹ denotes O or CH₂,
R' denotes alkyl or alkenyl having up to 8 carbon atoms,
L¹ and L², independently of one another, denote H or F,
L³ denotes H or F,
L⁴ denotes H or F, and
L⁵ and L⁶, independently of one another, denote H or CH₃.

2. Compounds according to Claim 1, **characterised in that**
L⁴ denotes H.

3. Compounds according to Claim 1 or 2, **characterised in that**
X¹ denotes F, OCF₃ or CF₃.

4. Compounds according to one or more of Claims 1 to 3, **characterised in that** L¹ and L² denote F.

5. Compounds according to one or more of Claims 1 to 4, **characterised in that** Y¹ denotes O.

6. Compounds according to one or more of Claims 1 to 4, **characterised in that** Y¹ denotes CH₂.

7. Compounds according to one or more of Claims 1 to 6 selected from the following formulae: 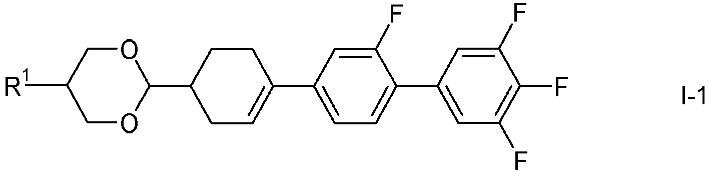 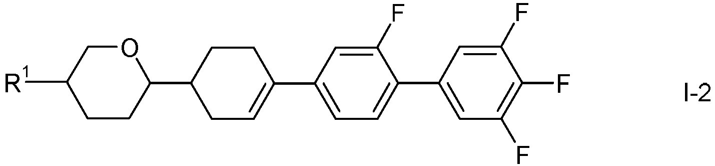 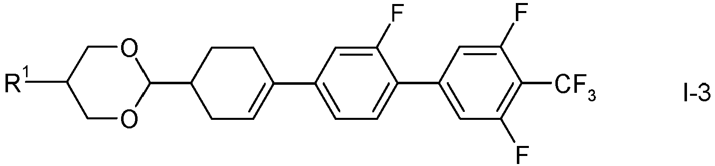 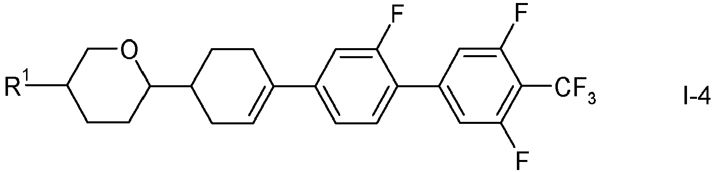 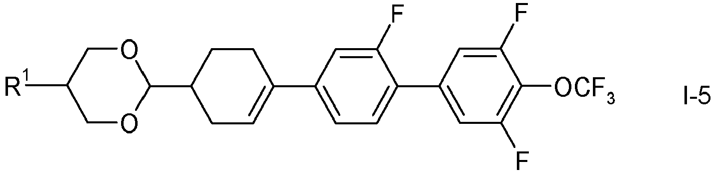 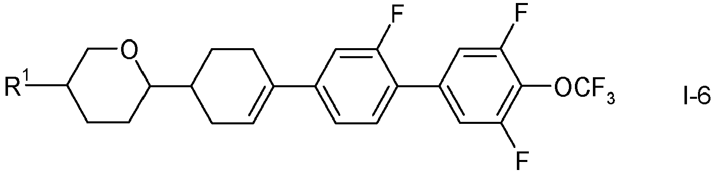 in which R' has the meaning indicated in Claim 1.

8. Compounds according to one or more of Claims 1 to 7, **characterised in that** R' denotes a straight-chain alkyl radical having 1 to 7 C atoms or an unbranched alkenyl radical having 2 to 8 C atoms.

9. Process for the preparation of compounds of the formula I according to one or more of Claims 1 to 8, including a process step in which an aryl-halogen compound of the formula II 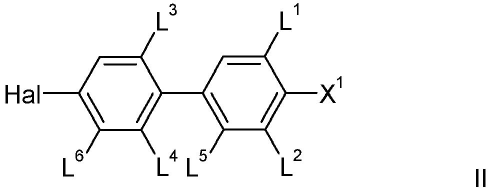 in which X¹, L¹, L², L³ and L⁴ are as defined in Claim 1, and
Hal denotes Br, I or Cl,
is reacted with a compound of the formula III 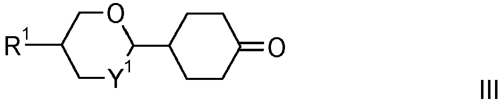
in which
R' and Y¹ are as defined in Claim 1,
to give a compound of the formula IV, 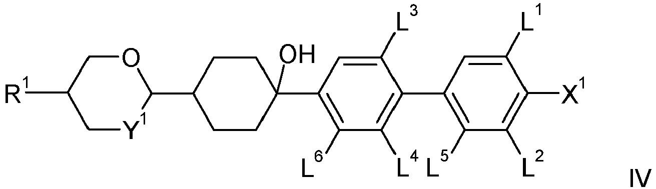
and is reacted further to give a compound of the formula I 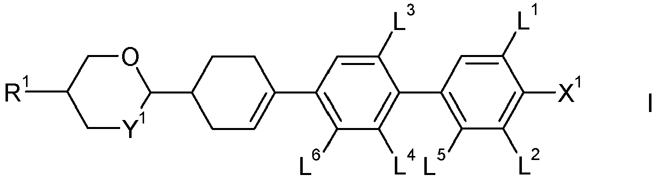 in which the groups R¹, Y¹, L¹, L², L³, L⁴ and X¹ are as defined in Claim 1.

10. Use of one or more compounds of the formula I according to one or more of Claims 1 to 8 as component in a liquid-crystalline medium.

11. Liquid-crystalline medium comprising at least two mesogenic compounds, **characterised in that** it comprises at least one compound of the formula I according to one or more of Claims 1 to 8.

12. Use of the liquid-crystalline medium according to Claim 11 for electro-optical purposes.

13. Electro-optical liquid-crystal display containing a liquid-crystalline medium according to Claim 11.

## Revendications

1. Composés de formule I, 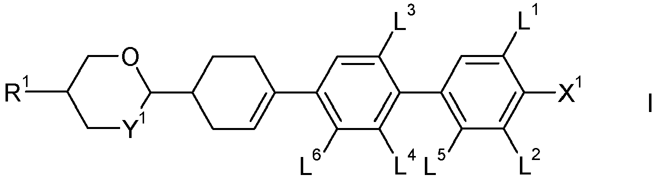 dans laquelle
X¹ désigne F, CF₃, OCF₃, OCHF₂, CHF₂, SCN ou CN,
Y¹ désigne O ou CH₂,
R' désigne alkyle ou alcényle ayant jusqu'à 8 atomes de carbone,
L¹ et L², indépendamment l'un de l'autre, désignent H ou F,
L³ désigne H ou F,
L⁴ désigne H ou F,
et
L⁵ et L⁶, indépendamment l'un de l'autre, désignent H ou CH₃.

2. Composés selon la revendication 1, **caractérisés en ce que**
L⁴ désigne H.

3. Composés selon la revendication 1 ou 2, **caractérisés en ce que**
X¹ désigne F, OCF₃ ou CF₃.

4. Composés selon l'une ou plusieurs parmi les revendications 1 à 3, **caractérisés en ce que** L¹ et L² désignent F.

5. Composés selon l'une ou plusieurs parmi les revendications 1 à 4, **caractérisés en ce que** Y¹ désigne O.

6. Composés selon l'une ou plusieurs parmi les revendications 1 à 4, **caractérisés en ce que** Y¹ désigne CH₂.

7. Composés selon l'une ou plusieurs parmi les revendications 1 à 6, choisis parmi les formules suivantes : 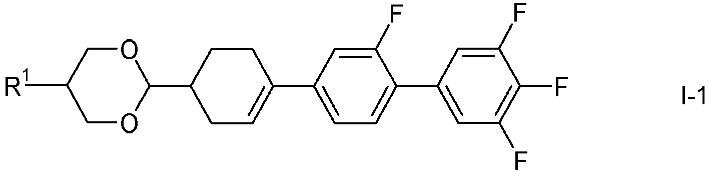 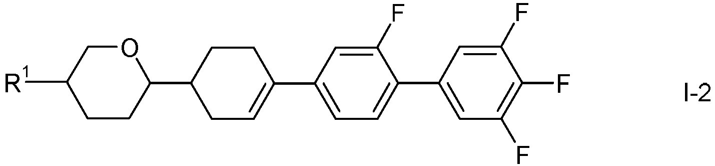 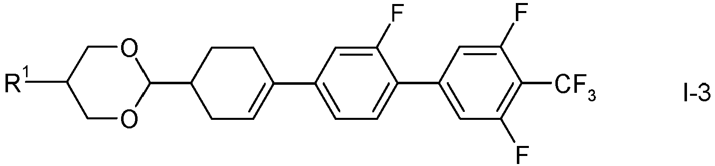 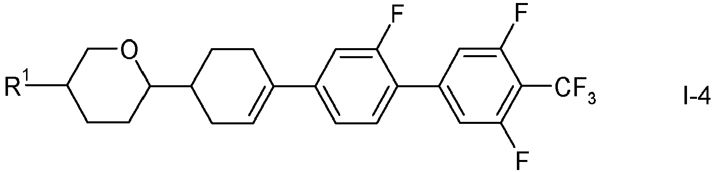 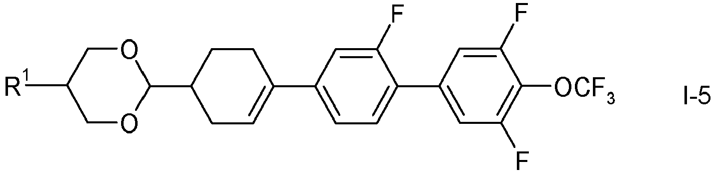 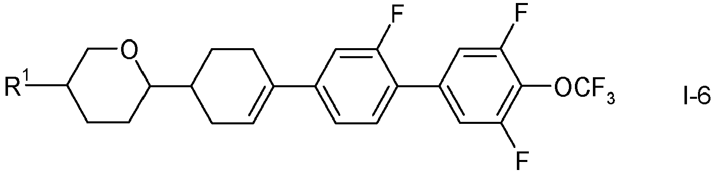 dans lesquelles R' revêt la signification indiquée selon la revendication 1.

8. Composés selon l'une ou plusieurs parmi les revendications 1 à 7, **caractérisés en ce que** R' désigne un radical alkyle à chaîne linéaire ayant de 1 à 7 atomes de C ou un radical alcényle non ramifié ayant de 2 à 8 atomes de C.

9. Procédé de préparation de composés de formule I selon l'une ou plusieurs parmi les revendications 1 à 8, comportant une étape de procédé dans laquelle un composé aryl-halogène de formule II 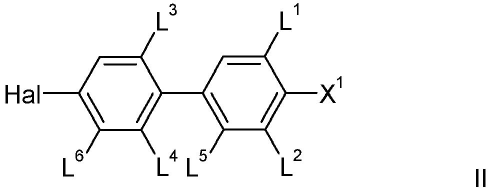 dans laquelle X¹, L¹, L², L³ et L⁴ sont tels que définis selon la revendication 1, et
Hal désigne Br, I ou Cl,
est réagi avec un composé de formule III 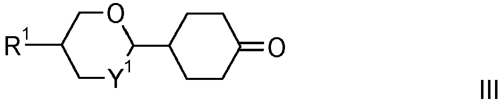 dans laquelle
R' et Y¹ sont tels que définis selon la revendication 1, pour donner un composé de formule IV, 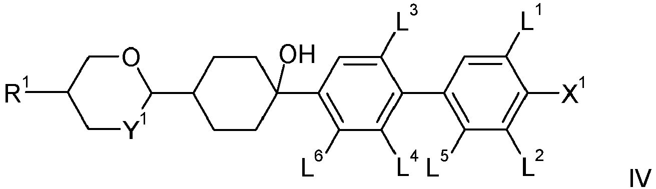
et est réagi davantage pour donner un composé de formule I 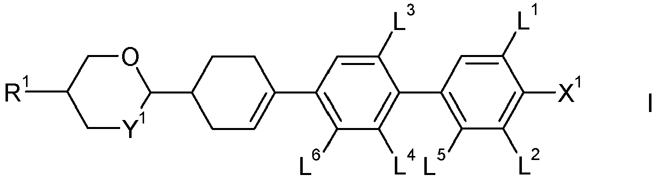
dans laquelle les groupements R¹, Y¹, L¹, L², L³, L⁴ et X¹ sont tels que définis selon la revendication 1.

10. Utilisation d'un ou plusieurs composés de formule I selon l'une ou plusieurs parmi les revendications 1 à 8, comme composant dans un milieu cristallin liquide.

11. Milieu cristallin liquide comprenant au moins deux composés mésogènes, **caractérisé en ce qu'**il comprend au moins un composé de formule I selon l'une ou plusieurs parmi les revendications 1 à 8.

12. Utilisation du milieu cristallin liquide selon la revendication 11, à des fins électro-optiques.

13. Affichage à cristaux liquides électro-optique, contenant un milieu cristallin liquide selon la revendication 11.
